# EUROPEAN PATENT APPLICATION

(11) **EP 4 180 069 A1**
(43) Date of publication of application: **17.05.2023**
(21) Application number: 21207960.2
(22) Date of filing: 12.11.2021
(51) Int. Cl.: A61M 5/142, A61M 5/145, A61M 5/168, A61M 5/172

(54) **SYSTEM FOR PUMPING A MEDICAL FLUID**

(71) Applicant: Fresenius Kabi Deutschland GmbH, 61352 Bad Homburg (DE)
(72) Inventor: Pütter, Harry, 36364 Bad Salzschlirf (DE)
(74) Representative: Fresenius Kabi Deutschland GmbH

(57) **Abstract**

A system (1) for pumping a medical fluid through a line in a medical pump (3) comprising: a pump segment (5) comprising an information storage means (15) comprising information pertaining to at least one individual characteristic of the pump segment (5); and
a medical pump (3) comprising a pumping chamber (11) for accommodating the pump segment (5), wherein the medical pump (3) further comprises a pump engine (13) and a reading means (17) for obtaining the information from the information storage means (15), and
processing means (19) adapted to adjust the pumping algorithm of the pump engine (13) based on the information. The invention also relates to a method (1000) for pumping a medical fluid through a line in a medical pump (3).

## Description

The invention relates to a system and method for pumping a medical fluid through a line in a medical pump.

Systems for pumping a medical fluid often employ positive displacement pumps (sometimes referred to as roller pumps) to pump the medical fluid contained within a flexible tube. A typical medical pump comprises a pumping chamber for accommodating a pump segment connected to the flexible tube or which is part of the flexible tube. Also, a typical medical pump further comprises a pump engine that is often connected to a rotor which typically carries at least one circumferential roller mounted on bearings, the roller is arranged to compress the pump segment and forcing the fluid to move through the pump segment in the direction of movement of the roller. The pump segment is fabricated from a resilient material and thus reassumes its normal shape after the compression by the roller ceases. A body of fluid (or bolus) trapped between two successive rollers is thus transported at ambient pressure toward the pump outlet.

Typically, such pumps are employed in the pumping of clean or sterile medical fluids, as there is no contact between the pump mechanism and the content of the tube. Such pumps are often used in medical applications, such as to pump IV fluids through infusion devices, in haemodialysis systems, or in heart-lung machines to circulate blood during bypass surgery. Such pumps are also often used to pump aggressive fluids and chemicals, including very viscous fluids and high solids slurries, where isolation of the material from the environment is important.

Pumps for pumping a medical fluid may run continuously, or they may be indexed through partial revolutions to deliver smaller amounts of fluid, and offer the advantages of low maintenance, few moving parts, prevention of backflow and siphon, and accurate dosing (as a fixed amount of fluid is pumped per rotation of the rotor). Therefore, the pump can, when calibrated, accurately produce a predefined flow rate. As an example, means for compensating for tolerance variations of pump components are described in EP 0 663 529 A1. Also, EP 2 822 640 A2 relates to calibrating housing assemblies.

Unfortunately, even with correctly calibrated pumps the current systems are limited in their dosing accuracy to the individual characteristics, e.g. individual dimensions, of the pump segment used in the system. Hence, the dosing accuracy depends on both the pump and the individual pump segment used with the pump. Pump segments are often manufactured as disposables in high numbers. Even though the pump segments are manufactured to stay within specific inherent tolerances, the pump segments are in most cases manufactured having dimensions greatly varying within their respective tolerance bands.

It is an object of the invention to provide a system for pumping a medical fluid where the individual characteristics of the pump segment are considered for increasing the systems overall performance characteristics.

This object is achieved by means of a system for pumping a medical fluid through a line in a medical pump comprising the features of claim 1.

Accordingly, the system comprises:
a pump segment comprising an information storage means comprising information pertaining to at least one individual characteristic of the pump segment; and
a medical pump comprising a pumping chamber for accommodating the pump segment,
wherein the medical pump further comprises a pump engine and a reading means for obtaining the information from the information storage means, and
processing means adapted to adjust the pumping algorithm of the pump engine based on the information.

The term "pump segment" herein is to be used in a broad manner. The pump segment is for example comprised in one of a tube element, a syringe or a medication cassette reservoir.

The term "pump segment" for example can be used herein to refer to an elastic means that can be at least partly displaced for pumping the medical fluid. In an example, the "pump segment" can be a section of a tube that is made from a resilient material and which is suitable to be used with the medical pump described herein. The pump segment can be a disposable sterile infusion tube having connection interfaces to connect the tube to an infusion line. In an another example, the term "pump segment" can be used to refer to an elastic membrane. For example, an elastic membrane is used if a medical fluid is pumped from a medication cassette reservoir.

The information storage means can be an optical marking, such as a laser marking, or an electronic marking, such as an RFID tag that comprises information pertaining to at least one individual characteristic of the pump segment. The individual characteristic can be a geometrical dimension of the pump segment that is currently used with the medical pump. Also, the individual characteristic can be a function of several geometrical dimensions of the pump segment. The geometrical dimension can be measured during the manufacturing process of each individual pump segment and can be stored on the information storage means of the corresponding pump segment for later use at the medical pump.

The medical pump can be a peristaltic pump, where the pumping action is based on at least one constriction or closure point (occlusion) moving along the length of the pump segment. The constriction or closure point can be realized as a movable rod, e.g as a finger element or as a roller element. The pump engine can be a motor, such as an electrical motor that is coupled to the moveable rod or roller element to drive the moveable rod or roller element.

The term "reading means" can be used to refer to means suitable for obtaining the information from the information storage means and that can be realized as an optical reader, such as a camera, or a reader suitable to detect a Radio Frequency, RF, signal such as a RFID reader.

The processing means adapted to adjust the pumping algorithm of the pump engine based on the information can be realized as a digital signal processing integrated circuit that is connected to the pump engine and adapted to control the pump engine.

The term "pumping algorithm" can be understood as an algorithm governing the application of system inputs to drive the system to a desired state. In particular, the pumping algorithm can be adjusted based on the information, such as setting an engine speed of the pump engine higher than an engine speed that would be used for a pump segment having "ideal" individual characteristics upon determining that the at least one individual characteristic would lead to a reduced flow rate of the medical fluid. Alternatively, the pumping algorithm can be adjusted by setting the engine speed of the pump engine lower than an engine speed that would be used for a pump segment having "ideal" individual characteristics upon determining that the at least one individual characteristic would lead to an increased flow rate of the medical fluid. Also, the term "adjusting the pumping algorithm" can be used herein to refer to selecting a specific pumping algorithm from a plurality of available pumping algorithms. The selected pumping algorithm can be a pumping algorithm which is most suitable, e.g. gives the most accurate results, for a specific individual characteristic of the pump segment. For example, a look-up table may be stored on the processing means that indicates which pumping algorithm is to be selected for each individual characteristic comprised in the information. It is to be understood that, if more than one individual characteristic of the pump segment is taken into account the look-up table is adjusted accordingly. The look up table may than be for example a 2D matrix.

Advantageously, adverse influences on the performance characteristics of the system for pumping a medical fluid caused by individual pump segments having dimensions randomly dispersed within a corresponding tolerance band can be reduced or even prevented by employing a system according to the invention.

In one example, the at least one individual characteristic comprises at least one geometrical dimension of the pump segment. The pump segment is referred to as a part of the system which interacts with the pump and which has due to its geometrical dimension an impact on the flow rate.
The at least one geometrical dimension can be a geometrical dimension that is suitable for influencing the inner volume of the pump segment that is pumped during one full cycle. For example, if a tube element is used, the length and diameter of the tube element could be relevant geometrical dimensions. Alternatively, if a medication cassette reservoir is used, geometrical dimensions such as the height, width and length of the medication cassette could be relevant geometrical dimensions.

In case more than one geometrical dimension for describing the inner volume is used, they have to be considered separately. A pump segment for example having the same inner volume but different length and different inner diameter do show different behavior during the pumping process. A corresponding look up table would use a matrix to take the geometrical dimension into account.

Advantageously, the geometrical dimension(s) can be measured, e.g. automatically measured during the manufacturing process, and stored as information in the information storage means.

In one example, the at least one geometrical dimension comprises an inner diameter of the pump segment, and/or a wall thickness of the pump segment, and/or an overall length of the pump-segment.

Advantageously, just one or a plurality of geometrical dimensions defining each pump segment can be stored on the information storage means.

In one example, adjusting the pumping algorithm comprises:
setting an engine speed of the pump engine to achieve an intended flow rate of the medical fluid.

Here, the term "intended flow rate" can be used to refer to an ideal flow rate that is achieved when an ideal pump segment, having ideal dimensions that are not varying within their respective tolerance bands, is used and an ideal engine speed is set, where the ideal engine speed is determined to achieve the ideal flow rate with the ideal pump segment.

For example, a value for a specific value for a specific pump segment having specific dimension can be obtained from a look-up table or matrix stored on the processing means. Depending on whether the at least one individual characteristic of the pump segment currently used in the system would lead to a higher or lower flow rate compared to the ideal flow rate when the ideal engine speed is set, the engine speed can be set lower or higher than the ideal engine speed.
Advantageously, by setting specific engine speeds corresponding to specific dimensions of the specific pump segments used in the system, a reproduceable flow rate can be achieved.

In one example, the pump segment comprises a first connecting interface on a first end to connect the pump segment to an upstream fluid line and a second connecting interface on a second end to connect the pump segment to a downstream fluid line to form a continuous fluid conduit, extending from a fluid supply comprised in a medication cassette reservoir, or a reservoir bag.

Advantageously, the pump segment can be a disposable product to which several types of fluid reservoirs can be connected to.

In one example, the information storage means comprises a Radio-Frequency Identification, RFID, tag and the reading means comprises a corresponding RFID reader.

Advantageously, an electromagnetic field can be used for automatically identifying the pump segment and to obtain the information. The RFID tag can be a passive tag that is powered by energy from the RFID reader's interrogating radio wave. Also, when using RFID technology, the RFID tag does not need to be within the line of sight of the reader, so it may be embedded, such as molded in the material of the pump segment. The information can then simply be obtained from the RFID tag when the pump segment is automatically inserted into the pumping chamber of the medical pump.

In one example, the information storage means comprises an optical marking arranged in the material of the pump segment, in particular a laser marking comprising a two-dimensional code, and the reading means is adapted to read the optical marking.

Advantageously, introducing an optical marking into the material of the pump segment can be realized in a cheap and efficient manner, since no additional parts, such as tags, for holding the information are necessary.

In one example, the pump segment is comprised in one of a tube element, a syringe or a medication cassette reservoir.

Advantageously, the pump segment can be used with a large number of reservoirs suitable for holding the medical fluid.

The invention is also concerned with a method for pumping a medical fluid through a line in a medical pump comprising the steps:
inserting a pump segment comprising an information storage means into a pumping chamber of the medical pump, wherein the information storage means comprises information pertaining to at least one individual characteristic of the pump segment;
recognizing, by a reading means of the medical pump, the information storage means and obtaining the information from the information storage means; and
adjusting, by a processing means of the medical pump, the pumping algorithm of a pump engine of the medical pump based on the information.

In one example, the at least one individual characteristic comprises at least one geometrical dimension of the pump segment.

In one example, the at least one geometrical dimension comprises an inner diameter of the pump segment, and/or a wall thickness of the pump segment, and/or an overall length of the pump-segment.

In one example, adjusting the pumping algorithm comprises:
setting an engine speed of the pump engine to achieve an intended flow rate of the medical fluid.

The idea underlying the invention shall subsequently be explained in more detail with reference to the embodiments shown in the figures. Herein:
- Fig. 1: shows a schematic drawing of an embodiment of a system for pumping a medical fluid; and
- Fig. 2: shows method steps of a method for pumping a medical fluid.

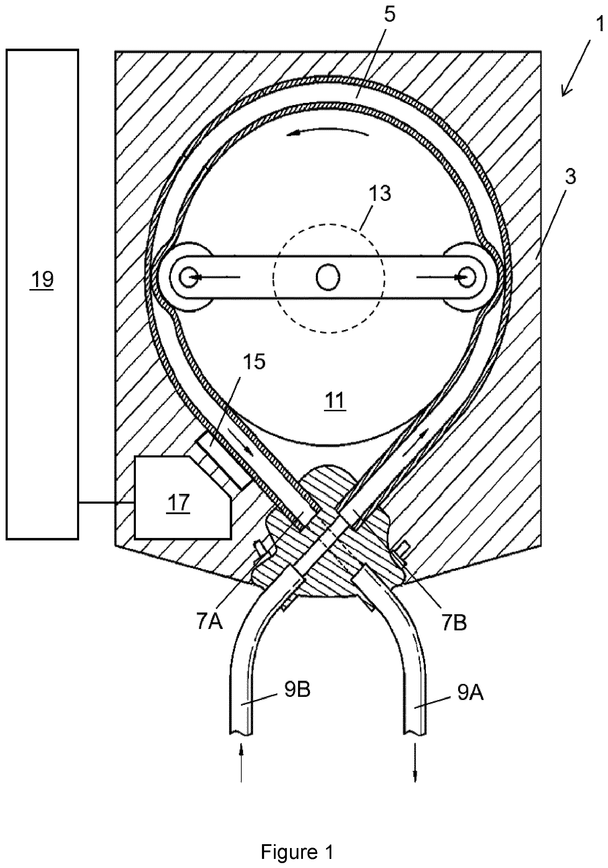

Fig. 1 shows a schematic drawing of a system 1 for pumping a medical fluid.

In the shown embodiment, the medical pump 3 comprised in the system 1 is shown as a positive displacement pump to pump the medical fluid contained within the pump segment 5, which is shown in figure 1 as a flexible tube arranged in a pumping chamber 11 of the medical pump 3. The pump engine 13 is connected to a rotor which carries two circumferential rollers mounted on bearings, each of which is arranged to compress the pump segment 5 and forcing the fluid to move through the pump segment 5 in the direction of movement of the rollers as indicated by the arrows shown in figure 1. The pump segment 5 is made from a resilient material and thus reassumes its normal calibre after the compression by the roller ceases. A body of fluid trapped between the two rollers is thus transported at ambient pressure from a second connecting interface 7B on a second end of the pump segment 5 that is connected to a downstream fluid line 9B extending from a fluid supply comprised in a medication cassette reservoir, or a reservoir bag (not shown in figure 1) towards a first connecting interface 7A on a first end of the pump segment 5 that is connected to an upstream fluid line 9A.

In the shown embodiment the pump segment 5 comprises an information storage means 15 comprising information pertaining to at least one individual characteristic of the pump segment 5. In the shown embodiment, the individual characteristic is at least one geometrical dimension suitable for describing the inner volume of the pump segment that is pumped during one full cycle, such as the inner diameter and length of the pump segment 5 for calculating the inner volume at the processing means 19. The geometrical dimension can be measured during the manufacturing process of the pump segment 5 and can be stored on the information storage means 15 of the pump segment 5.

The shown information storage means 15 is realized as a Radio-Frequency Identification, RFID, tag and the reading means 17 is realized as a corresponding RFID reader for reading the information from the information storage means 15.

In other embodiments the information storage means can also comprise an optical marking arranged in the material of the pump segment, such as a laser marking comprising a two-dimensional code, and the reading means, for example a camera, is adapted to read the optical marking. In further embodiments the optical marking could be also realized as QR code and the corresponding reading means as a corresponding scanning device.

Also, the information storage means 15 and the reading means 17 can be located at different locations in the system 1, i.e. at locations different than shown in figure 1.

The processing means 19 which is adapted to adjust the pumping algorithm of the pump engine 13 based on the information is shown in the embodiment outside the housing of the medical pump 3. In alternative embodiments, the processing means 19 can be also located inside the housing of the medical pump 3. The processing means 19 is connected to the pump engine 13 and is adapted to control the pump engine 13, such as to control the speed of the pump engine 13.

The pumping algorithm is stored in the processing means 19 and is adjusted based on the information. Adjusting the pumping algorithm comprises setting an engine speed of the pump engine 13 to achieve an intended flow rate of the medical fluid. A value for a specific value for a specific pump segment having specific dimension is obtained from a look-up table or matrix stored on the processing means. Depending on whether the at least one individual characteristic of the pump segment currently used in the system would lead to a higher or lower flow rate compared to the ideal flow rate when the ideal engine speed is set, the engine speed is set lower or higher than the ideal engine speed..

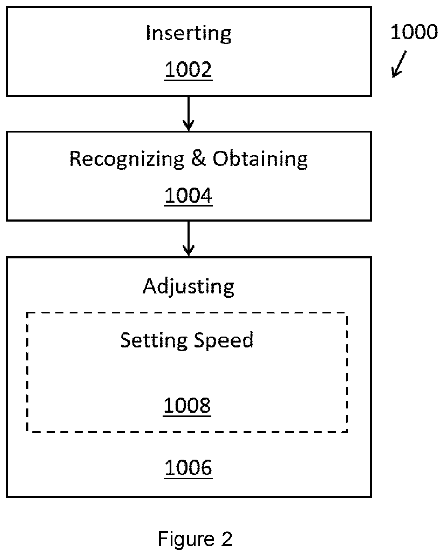

Figure 2 shows method steps of a method 1000 for pumping a medical fluid.

The method 1000 for pumping the medical fluid through a line in a medical pump comprising the steps:
Inserting 1002 a pump segment comprising an information storage means into a pumping chamber of the medical pump, wherein the information storage means comprises information pertaining to at least one individual characteristic of the pump segment;
Recognizing 1004, by a reading means of the medical pump, the information storage means and obtaining the information from the information storage means; and
Adjusting 1006, by a processing means of the medical pump, the pumping algorithm of a pump engine of the medical pump based on the information.

The step of Adjusting 1006 can comprise the optional step of:
Setting 1008 an engine speed of the pump engine to achieve an intended flow rate of the medical fluid.

### List of Reference Numerals

- 1: System for Pumping a Medical Fluid
- 3: Medical Pump
- 5: Pump Segment
- 7A: First Connecting Interface
- 7B: Second Connecting Interface
- 9A: Upstream Fluid Line
- 9B: Downstream Fluid Line
- 11: Pumping Chamber
- 13: Pump Engine
- 15: Information Storage Means
- 17: Reading Means
- 19: Processing Means

- 1000: Method for Pumping a Medical Fluid
- 1002: Inserting
- 1004: Recognizing
- 1006: Adjusting
- 1008: Setting Engine Speed

## Claims

1. A system (1) for pumping a medical fluid through a line in a medical pump (3) comprising:
a pump segment (5) comprising an information storage means (15) comprising information pertaining to at least one individual characteristic of the pump segment (5); and
a medical pump (3) comprising a pumping chamber (11) for accommodating the pump segment (5), wherein the medical pump (3) further comprises a pump engine (13) and a reading means (17) for obtaining the information from the information storage means (15), and
processing means (19) adapted to adjust the pumping algorithm of the pump engine (13) based on the information.

2. System (1) according to claim 1, **characterized in that** the at least one individual characteristic comprises at least one geometrical dimension of the pump segment (5).

3. System (1) according to claim 2, **characterized in that** the at least one geometrical dimension comprises an inner diameter of the pump segment (5), and/or a wall thickness of the pump segment (5), and/or an overall length of the pump-segment (5).

4. System (1) according to any of the preceding claims, **characterized in that** to adjust the pumping algorithm comprises:
setting an engine speed of the pump engine to achieve an intended flow rate of the medical fluid.

5. System (1) according to any of the preceding claims, **characterized in that** the pump segment (5) comprises a first connecting interface (7A) on a first end to connect the pump segment (5) to an upstream fluid line (9A) and a second connecting interface (7B) on a second end to connect the pump segment (5) to a downstream fluid line (9B) to form a continuous fluid conduit, extending from a fluid supply comprised in a medication cassette reservoir, or a reservoir bag.

6. System (1) according to any of the preceding claims, **characterized in that** the information storage means (15) comprises a Radio-Frequency Identification, RFID, tag and the reading means (17) comprises a corresponding RFID reader.

7. System (1) according to any of the preceding claims, **characterized in that** the information storage means (15) comprises an optical marking arranged in the material of the pump segment (5), in particular a laser marking comprising a two-dimensional code, and the reading means (17) is adapted to read the optical marking.

8. System (1) according to any of the preceding claims, **characterized in that** the pump segment (5) is comprised in one of a tube element, a syringe or a medication cassette reservoir.

9. A method (1000) for pumping a medical fluid through a line in a medical pump (3) comprising the steps:
inserting (1002) a pump segment (5) comprising an information storage means (15) into a pumping chamber (11) of the medical pump (3), wherein the information storage means (15) comprises information pertaining to at least one individual characteristic of the pump segment (5);
recognizing (1004), by a reading means (17) of the medical pump (3), the information storage means (15) and obtaining the information from the information storage means (15); and
adjusting (1006), by a processing means (19) of the medical pump (3), the pumping algorithm of a pump engine (13) of the medical pump (3) based on the information.

10. Method according to claim 9, **characterized in that** the at least one individual characteristic comprises at least one geometrical dimension of the pump segment (5).

11. Method according to claim 10, **characterized in that** the at least one geometrical dimension comprises an inner diameter of the pump segment (5), and/or a wall thickness of the pump segment, and/or an overall length of the pump-segment (5).

12. Method according to any of the preceding claims, **characterized in that** adjusting the pumping algorithm comprises:
setting (1008) an engine speed of the pump engine to achieve an intended flow rate of the medical fluid.
